# EUROPEAN PATENT SPECIFICATION

(11) **EP 4 025 042 B1**
(45) Date of publication and mention of the grant of the patent: **04.11.2026**
(21) Application number: 20860848.9
(22) Date of filing: 04.09.2020
(51) Int. Cl.: A61K 31/194, A61K 38/46, A61K 45/06, A61L 15/46, A61P 17/02, C12N 9/22

(54) **NOVEL COMPOSITIONS FOR DISRUPTING BIOFILMS**
NEUARTIGE ZUSAMMENSETZUNGEN ZUR ZERSTÖRUNG VON BIOFILMEN
NOUVELLES COMPOSITIONS DE RUPTURE DE BIOFILMS

(30) Priority: 06.09.2019 US 201962896767 P; 10.09.2019 US 201962898205 P
(43) Date of publication of application: 13.07.2022
(73) Proprietor: The Trustees of Indiana University, Bloomington, IN 47405 (US)
(72) Inventor: SEN, Chandan K., Indianapolis, Indiana 46202 (US); GHATAK, Subhadip, Indianapolis, Indiana 46202 (US)
(74) Representative: HGF
(86) International application number: PCT/US2020/049433
(87) International publication number: WO 2021/046369

(56) References cited:
- EP-B1- 3 127 582
- WO-A2-2012/106264
- US-A1- 2011 086 101
- US-A1- 2013 236 920
- US-A1- 2014 329 780
- US-B2- 10 323 217
- US-B2- 7 998 699
- US-B2- 9 675 736
- BAELO AIDA ET AL: "Disassembling bacterial extracellular matrix with DNase-coated nanoparticles to enhance antibiotic delivery in biofilm infections", JOURNAL OF CONTROLLED RELEASE, ELSEVIER, AMSTERDAM, NL, vol. 209, 23 April 2015 (2015-04-23), pages 150 - 158, XP029173934, ISSN: 0168-3659, DOI: 10.1016/J.JCONREL.2015.04.028

## Description

### CROSS REFERENCE TO RELATED APPLICATIONS

This application claims priority to the following: U.S. Provisional Patent Application No. 62/896,767 filed on September 6, 2019 and U.S. Provisional Patent Application No. 62/898,205 filed on September 10, 2019.

### BACKGROUND OF THE DISCLOSURE

A biofilm represents a group of microorganisms in which cells stick to each other to form an aggregation of cells, and often the aggregation of cells adhere to a surface. These adherent cells are typically embedded within a self-produced matrix of extracellular polymeric substance (EPS). Biofilms cause a significant amount of all human microbial infections. Biofilm formation and persistence has profound implications for the patient, because microorganisms growing as biofilms are significantly less susceptible to antibiotics and host defenses and they commonly manifest as chronic or recurrent infections. Biofilm infections constitute a number of clinical challenges, including diseases involving uncultivable species, chronic inflammation, impaired wound healing, and rapidly acquired antibiotic resistance.

The biofilm EPS is typically comprised of a polymeric conglomeration generally composed of extracellular DNA, proteins, and polysaccharides. Biofilms may form on living or non-living surfaces and can be prevalent in natural, industrial and hospital settings.

Biofilms are highly resistant to antibiotics and host immune defenses in part due to their structural and phenotypic characteristics. The extracellular polymeric substance (EPS) plays a pivotal role in the structural organization of biofilms. In addition to reinforcing the physical strength of biofilm, EPSs also promote microbial interaction and communication, enhance horizontal gene transfer, trap nutrients, and even provide nutrients to the persistent bacteria. Accordingly, due in part to the ability of microbes in biofilm to escape recognition by the host immune cells or eradication by antibiotics, biofilms cause a significant amount of all human microbial infections.

A growing body of research now acknowledges the presence of extracellular forms of DNA and their role as important structural components of the biofilm matrix. The use of enzymes, including nucleases, to help disrupt biofilms has been suggested as a potential treatment for treating biofilms and disrupting aggregations of pathogenic cells. WO 2012/106264 describes a pharmaceutical composition for inhibiting biofilm formation, thereby treating or preventing bacterial vaginosis. The composition is topically administered and comprises DNase and an antibiotic.

However, subpopulations of biofilms have been encountered that are resistant to nuclease treatments and represent a persistent subgroup. Accordingly, additional therapeutic compositions are desired that are effective in disrupting the aggregation of these persistent biofilm populations.

Disclosed herein are compositions and said compositions for use in methods for degrading biofilms that are resistant to standard DNase treatments. The compositions disclosed herein can be used in conjunction with standard techniques for removing and/or killing microorganisms associated with biofilms. According to the claimed invention, a biofilm degrading composition is provided comprising a DNase that has endonuclease or exonuclease activity, aurine tricarboxylic acid (ATA) that disrupts protein-nucleic acid interactions, an anti-microbial agent, and a pharmaceutically acceptable carrier.

### SUMMARY

The scope of this invention is defined by the claims. Any references in the description to methods of treatment refer to the biofilm disrupting compositions of the present invention for use in a method for treatment of the human (or animal) body by therapy. Any "embodiment" or "example" which is disclosed in the description but is not covered by the claims should be considered as presented for illustrative purpose only.

Aggregation of pathogenic organisms enhances microbial pathogenicity by hindering host defenses and reducing the susceptibility of the pathogen to antibiotics. The presence of microorganisms growing as biofilms in wounds commonly manifest as chronic or recurrent infections and their presence interferes with wound closure. While enzymatic treatments have been suggested for disrupting the extracellular polymeric substance (EPS) comprising the matrix of the biofilm, hyperbiofilm variants are known that exhibit resistance to enzymatic treatment of the biofilms. However, in accordance with one embodiment of the present disclosure, compositions are provided comprising a DNase that hydrolyses nucleic acids, aurine tricarboxylic acid (ATA) that disrupts the binding of nucleic acids to proteins, an anti-microbial agent, and a pharmaceutically acceptable carrier. Such compositions are capable of disaggregating biofilms including hyperbiofilm variants resistant to disaggregating by treatment with enzymes alone.

Optionally the DNase is selected from the group consisting of Deoxyribonuclease I (DNase I), Deoxyribonuclease II (DNase II), Deoxyribonuclease **III** (DNase III), and micrococcal nuclease.

More particularly, in one embodiment the DNase I is a protease-free DNase I stable at pH 5-7 for at least five hours that is capable of high activity at low pH.

In one embodiment the composition for disrupting biofilms is formulated as an ointment, a gel, a liquid, an aerosol, a mist, a film, an emulsion, or a suspension. In one embodiment the composition is formulated as a gel. Such formulations comprising ATA, anda nuclease, are suitable for direct contact with biofilms to disrupt cellular aggregation and assist in the removal and/or termination of the associated pathogenic organism. In particular, the formulations can be used to treat hyperbiofilm variants of bacteria that are resistant to standard DNase treatment, including use for the topical treatment of infected chronic wounds or as a prophylactic treatment for any wound. According to the claimed invention the composition further comprises one or more anti-microbial agents, including for example antibiotics or antifungal agents.

In one embodiment the composition for disrupting biofilms further comprise additional enzymes for hydrolyzing polymers other than nucleic acids. In one embodiment the compositions comprises ATA and a DNase, and one or more additional enzymes selected from the group consisting of an amylase, cellulase, and a protease, or mixtures thereof.

**The description describes a** method for treating a biofilm infection wherein the biofilm is contacted with a composition comprising **ACA and a nuclease.**

In one embodiment the biofilm comprises one or more microorganisms selected from the group consisting of *Staphylococcusaureus, Staphylococcus epidermidis, Streptococcus sp., mycobacterium tuberculosis, Klebsiella pneumonia, Pseudomonas aeruginosa, Candida sp.,* and *Candida albicans.* In one embodiment the biofilm is a polymicrobial biofilm wherein the polymicrobial population comprises two or more different species of microorganisms. In one embodiment the polymicrobial biofilm comprises organisms selected from bacterial and fungal microorganisms.

**The description describes a method** for adversely affecting an established biofilm wherein the method comprising contacting the biofilm with a composition **comprising an effective amount of a nuclease and aurine tricarboxylic acid (ACA).**

**The description describes** the biofilm is present on the wounded surfaces of mammalian skin.

### BRIEF DESCRIPTION OF THE DRAWINGS

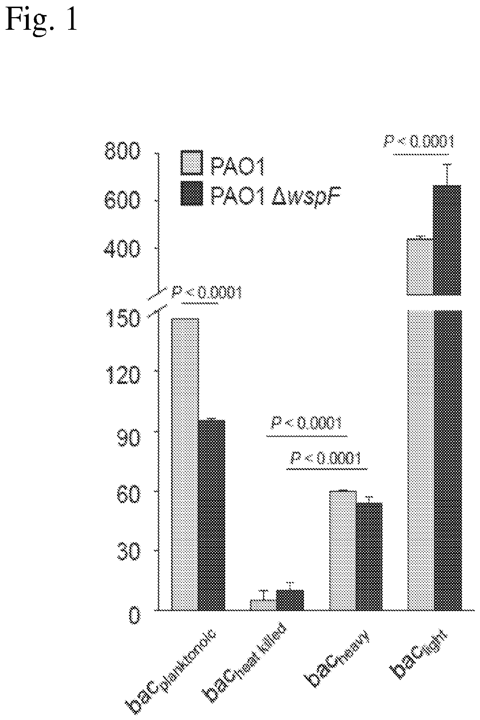

Fig. 1 is a bar graph providing data relating to real-time changes in oxygen consumption rate (OCR, in picomoles of molecular oxygen per minute) measured on a Seahorse XFe Extracellular Flux Analyzer in bac_{light} fraction and bac_{heavy} fractions of *P. aeruginosa* PAO1 and PAO1ΔwspF biofilms. (n = 10). Data are mean ± SD. The designations of bac_{light} fraction and bac_{heavy} fraction represent two difference subpopulations of bacteria based on differences detected in density. Unlike bacteria present in a PAOT1 biofilm where the bac_{light} and bac_{heavy} cells were homogenously distributed throughout the biofilm, bac_{light} and bac_{heavy} cells are segregated in the PAO1ΔwspF biofilms (data not shown).

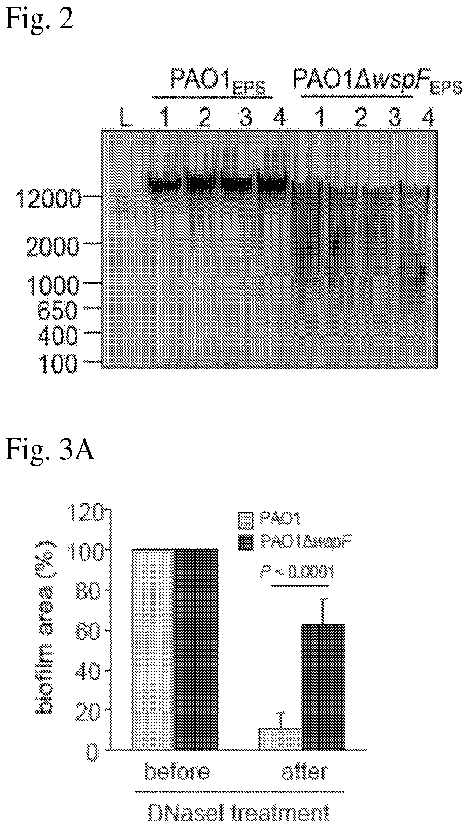

Fig. 2 is a photograph of an agarose gel electrophoresis of the DNA isolated from the EPS of *P. aeruginosa* PAO1 and PAO1ΔwspF biofilms. The data presented demonstrates that eDNA is mainly intact in DNA isolated from the EPS of the PAO1 biofilm. However, the DNA isolated from the EPS of the PAO1ΔwspF biofilms is fragmented.

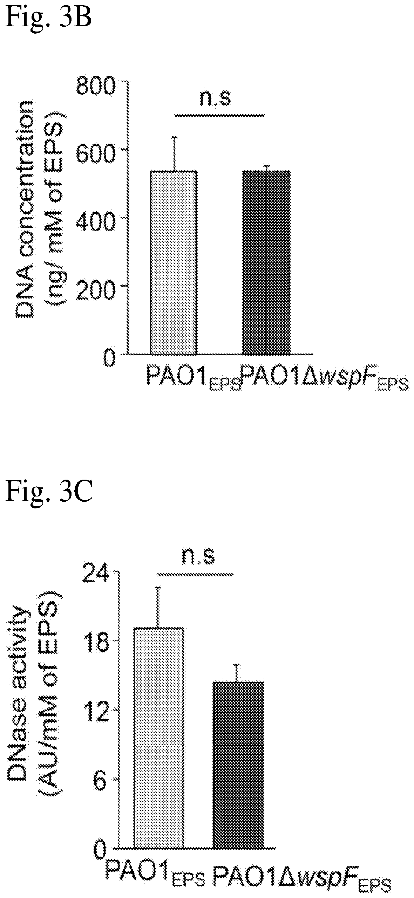

Fig. 3A-3C: Digital microphotographs of PAO1 and PAO1ΔwspF biofilms were taken before and after DNase I treatment. The areas of the biofilm before and after DNase I treatment were quantified using image J and expressed graphically as shown in Fig. 3A (n=6) demonstrating PAO1ΔwspF biofilms are more resilient than PAO1 biofilms to such treatments. Quantification of eDNA (Fig. 3B) and DNase activity (Fig. 3C) from the EPS of PAO1 and PAO1ΔwspF biofilms showed no significant difference in the eDNA content and DNase activity between the two biofilms. (n = 4) Data are mean ± SD. However, eDNA in the PAO1ΔwspF biofilm was found to consist of only part of PAO1ΔwspF genomic DNA.

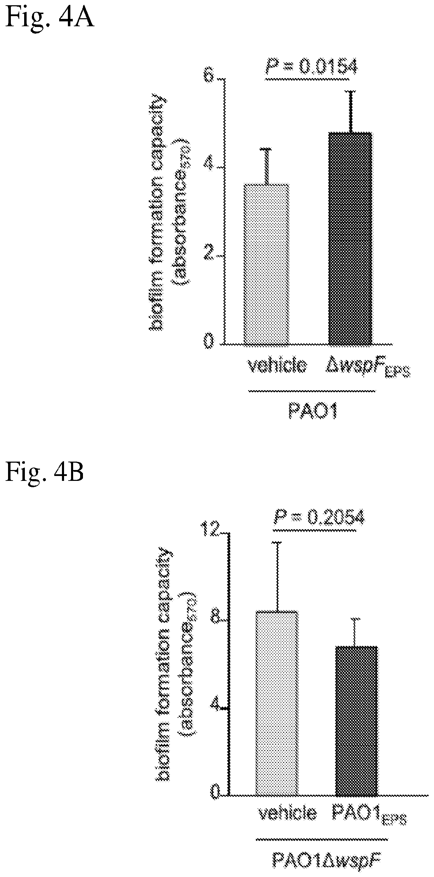

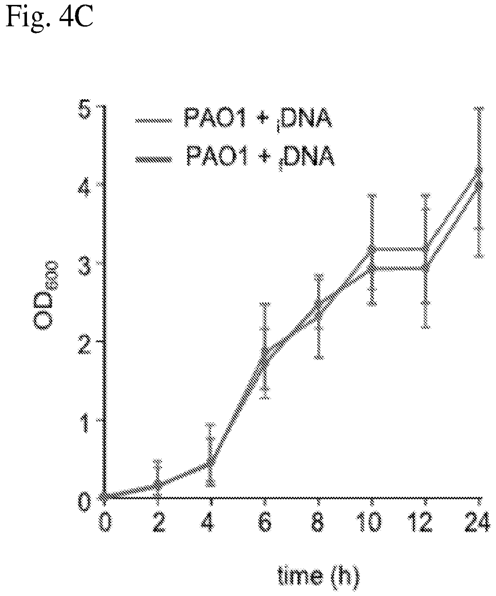

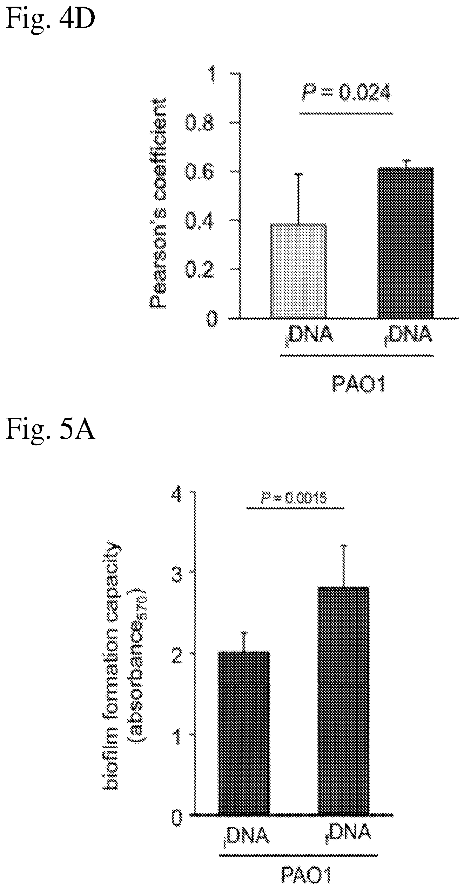

Figs. 4A-4D Interaction of Fragmented DNA with EPS Protein Results in Formation of Robust Biofilm. Fig. 4A is a bar graph presenting data from a crystal violet assay of PAO1 hydrated biofilm at 12 h treated with 1 ul of PAO1ΔwspF EPS (2 ug of eDNA) (n= 8) Fig. 4B is a bar graph presenting data from a crystal violet assay of PAO1ΔwspF hydrated biofilm at 24 h treated with 1 ul of PAO EPS (2 ug of eDNA) (n= 8). Fig. 4C is a graph showing the growth curve of PAO1 that were treated with intact genomic DNA (iDNA) and fragmented genomic DNA (fDNA) isolated from PAO1. Fig. 4D provides a graph showing the Pearson's coefficient from Fig. 4B of the PAO1 biofilm at 12h treated with intact genomic DNA (iDNA) and fragmented genomic DNA (fDNA) were plotted graphically as mean ± SD (n=4).

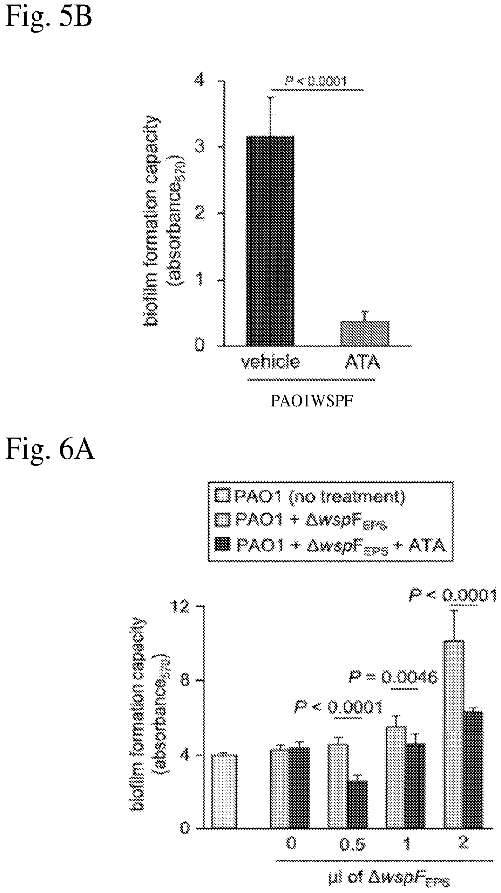

Figs. 5A & 5B: Fig. 5A is a bar graph presenting data from a crystal violet assay of PAO1 biofilm at 12 h treated with 500 ng intact genomic DNA (iDNA), and fragmented genomic DNA (fDNA) (n - 8). Data are mean ± SD. Fig. 5B is a bar graph presenting data from a crystal violet assay of PAO1ΔwspF biofilm at 24 h treated with buffer and ATA (n - 8). Inhibition of DNA-protein interaction compromised *in vitro* PAO1ΔwspF biofilm formation. Data are mean ± SD.

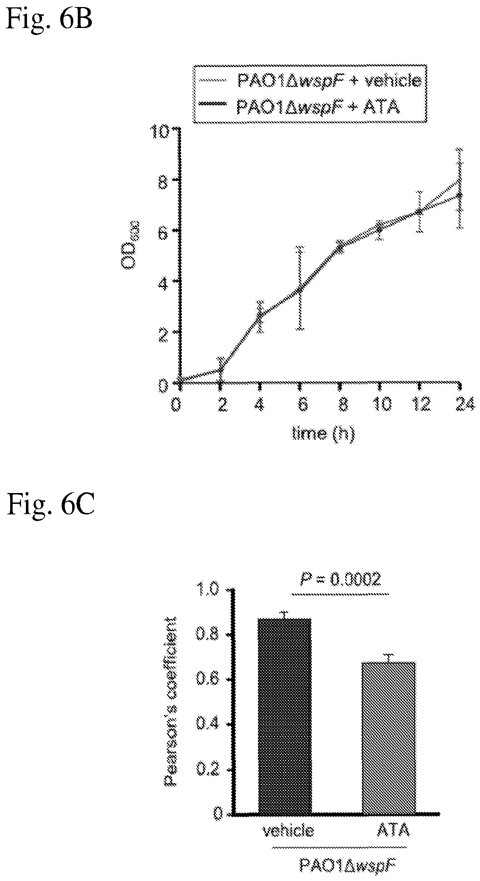

Figs. 6A-6C: Effect of ATA on PA01ΔwspF biofilm. Crystal violet assay of untreated PAO1 hydrated biofilm at 12h and PAO1 hydrated biofilm treated with different volumes of PAO1ΔwspF EPS (1µ1 = 2ug of eDNA) in presence of 0.5µM of ATA (n=8). Inhibition of DNA-protein interaction compromised *in vitro* PAO1 biofilm formation. The growth curve of PAO1ΔwspF biofilm treated with buffer and ATA is shown in Fig. 6B demonstrating ATA did not affect cell growth. The Pearson's coefficient of the PAO1ΔwspF biofilm at 24h treated with vehicle and ATA were plotted graphically as mean ± SD (n=4) and the data presented in Fig. 6C.

### DETAILED DESCRIPTION

### DEFINITIONS

**In** describing and claiming the invention, the following terminology will be used in accordance with the definitions set forth below.

As used herein, the term "pharmaceutically acceptable carrier" includes any of the standard pharmaceutical carriers, such as a phosphate buffered saline solution, water, emulsions such as an oil/water or water/oil emulsion, and various types of wetting agents. The term also encompasses any of the agents approved by a regulatory agency of the US Federal government or listed in the US Pharmacopeia for use in animals, including humans.

As used herein the term "pharmaceutically acceptable salt" refers to salts of compounds that retain the biological activity of the parent compound, and which are not biologically or otherwise undesirable. Many of the compounds disclosed herein are capable of forming acid and/or base salts by virtue of the presence of amino and/or carboxyl groups or groups similar thereto.

As used herein, the term "treating" includes prophylaxis of the specific disorder or condition, or alleviation of the symptoms associated with a specific disorder or condition and/or preventing or eliminating said symptoms.

As used herein an "effective" amount or a "therapeutically effective amount" of an biofilm disrupting composition refers to a nontoxic but sufficient amount of the composition to provide the desired effect, which in the case of the present invention is to adversely affect a biofilm. The exact amount required to achieve the desired result will vary depending on various factors such as a subject or a situation under consideration, the composition of the biofilm, the volume or size of the biofilm to be exposed to the composition, the environment in which the biofilm is located and the means by which exposing the biofilm to the composition is conducted. An effective amount can be provided for in one or more applications, administrations or dosages and is not intended to be limited to a particular formulation, administration route or application method. Accordingly, it is not practical to specify an exact "effective amount". Taking into account the particular circumstances, a person skilled in the art could readily determine the "effective amount" through routine experimentation.

As used herein, the term "purified" and like terms relate to the isolation of a molecule or compound in a form that is substantially free of contaminants normally associated with the molecule or compound in a native or natural environment. As used herein, the term "purified" does not require absolute purity; rather, it is intended as a relative definition. The term "purified RNA" is used herein to describe an RNA sequence which has been separated from other compounds including, but not limited to polypeptides, lipids and carbohydrates.

The term "isolated" requires that the referenced material be removed from its original environment (e.g., the natural environment if it is naturally occurring). For example, a naturally-occurring nucleic acid present in a living animal is not isolated, but the same nucleic acid, separated from some or all of the coexisting materials in the natural system, is isolated.

As used herein the term "patient" without further designation is intended to encompass any warm blooded vertebrate domesticated animal (including for example, but not limited to livestock, horses, mice, cats, dogs and other pets) and humans receiving a therapeutic treatment, self-administered or otherwise.

As used herein the term "solid support" relates to a solvent insoluble substrate that is capable of forming linkages (preferably covalent bonds) with soluble molecules. The support can be either biological in nature, such as, without limitation, a cell or bacteriophage particle, or synthetic, such as, without limitation, an acrylamide derivative, glass, plastic, agarose, cellulose, nylon, silica, or magnetized particles. The support can be in particulate form or a monolythic strip or sheet. The surface of such supports may be solid or porous and of any convenient shape.

As used herein, the term "parenteral" includes administration subcutaneously, intravenously or intramuscularly.

As used herein the term "nuclease" is defined as any enzyme that can cleave the phosphodiester bonds between nucleotides of nucleic acids. The term encompasses both DNases and RNases that effect single or double stranded breaks in their target molecules. A DNase is a nuclease that catalyzes the hydrolytic cleavage of phosphodiester linkages in a DNA backbone, whereas an RNase is a nuclease that catalyzes the hydrolytic cleavage of phosphodiester linkages in an RNA backbone. The nuclease may be indiscriminate about the DNA/RNA sequence at which it cuts or alternatively, the nuclease may be sequence-specific. The nuclease may cleave only double-stranded nucleic acid, only single-stranded nucleic acid, or both double-stranded and single stranded nucleic acid. The nuclease can be an exonuclease, that cleaves nucleotides one at a time from the end of a polynucleotide chain or an endonuclease that cleaves a phosphodiester bond within a polynucleotide chain. Deoxyribonuclease I (DNase I) is an example of a DNA endonuclease that cleaves DNA (causing a double stand break) relatively nonspecifically in DNA sequences.

As used herein the term "cellulase" is defined as any enzyme, or group of enzymes, that hydrolyze cellulose. Cellulose is a linear polysaccharide of glucose residues connected by β-1,4 linkages.

As used herein the term "amylase" is defined as any enzyme that hydrolyze glycosidic bonds found in polysaccharides such as starch.

As used herein the term "protease" is defined as any enzyme that hydrolyze peptide bonds found in proteins.

As used herein an antimicrobial is any agent that kills microorganisms or stops their growth, including microorganisms selected from the group consisting of bacteria, protists, and fungi.

The term "biofilm" as used herein means a community of one or more microorganisms attached to a surface, with the organisms in the community being contained within an extracellular polymeric substance (EPS) matrix produced by the microorganisms. **In** one embodiment the microorganism is a bacterial organism. I one embodiment the biofilm is polymicrobial, containing two or more different microorganisms.

The expression "biofilm forming microorganism" encompasses any microorganism that is capable of forming a biofilm, including monomicrobial and polymicrobial biofilms.

The terms "attached" and "adhered" when used in reference to bacteria or a biofilm and a surface means that the bacteria and biofilm are established on, and at least partially coats or covers the surface, and has some resistance to removal from the surface. No particular mechanism of attachment or adherence is intended by such usage.

The terms "detaching" or "removing" when used in reference to bacteria or a biofilm that is attached to a surface encompasses any process wherein a significant amount (for example at least 40%, 50%, 60%, 70%, 80% or 90%) of the bacteria or biofilm initially present on the surface is no longer attached to the surface.

As used herein the phrase "disrupting a biofilm" defines a process wherein the biofilm has been physically modified in a manner that increases the ease of detaching or removing the microorganisms comprising the biofilm through the use of standard procedures.

As used herein the term "adversely affecting" a biofilm, or a biofilm being "adversely affected" is intended to mean that the viability of the biofilm is compromised in some way. For example, a biofilm will be adversely affected if the number of live microorganisms that form part of the biofilm is reduced. A biofilm may also be adversely affected if its growth is inhibited, suppressed, or prevented.

### EMBODIMENTS

Biofilms can be establish on a wide range of surfaces and have been associated with many pathogenic forms of human diseases and plant infections. A growing body of research now acknowledges the presence of extracellular forms of DNA (eDNA) and their role as important structural components of the biofilm matrix. The use of enzymes, including nucleases, to help disrupt biofilms has been suggested as a potential treatment for biofilms to disrupt aggregations of pathogenic cells. However, subpopulations of biofilms have been encountered that are resistant to nuclease treatments and represent a persistent subgroup having "hyperbiofilm" characteristics. As indicated by the data presented in Example 1 bacteria with hyperbiofilm characteristics have been found to employ fragmented eDNA to achieve better interaction with macromolecules in the EPS.

Disclosed herein are compositions and methods for degrading biofilms and more particularly, disrupting established biofilms that are resistant to standard DNase treatments. Applicant has discovered that the inclusion of biocompatible agents that disrupt non-covalent bonding between nucleic acids and proteins can be effective in promoting the disruption of hyperbiofilms that are resistant to conventional treatments. The compositions disclosed herein can be used in conjunction with any standard techniques for removing and/or killing microorganisms associated with biofilms. Accordingly, therapeutic compositions are provided herein that are effective in disrupting the aggregation of these persistent biofilm populations.

In accordance with one embodiment a biofilm disrupting composition is provided comprising a biocompatible agent that disrupts non-covalent bonding between nucleic acids and proteins, wherein the agent is aurine tricarboxylic acid (ATA).

In one embodiment such compositions can be formulated for topical administration, including for example formulated as a gel comprising ATA. The compositions comprising ATA are further combined with a DNase that has endonuclease or exonuclease activity, and optionally further enzymes that hydrolyze polymeric compounds, including for example proteases, amylases and cellulases.

The composition further comprises an anti-microbial agent and a pharmaceutically acceptable carrier. In one embodiment the DNase of the biofilm disrupting composition is selected from the group consisting of Deoxyribonuclease I (DNase I), Deoxyribonuclease II (DNase II), Deoxyribonuclease III (DNase III), micrococcal nuclease, and a recombinant DNase. In one embodiment the nuclease is DNase I.

The biofilm disrupting compositions disclosed herein can be combined with standard pharmaceutically acceptable carriers. In one embodiment the composition is formulated as an ointment, a gel, a liquid, an aerosol, a mist, a film, an emulsion, or a suspension. In one embodiment the formulation is prepared for sustained extended release of the active agents using standard formulations. In one embodiment the composition is formulated as a topical formulation for application to mammalian skin, optionally for contact with wounded skin tissue. In accordance with one embodiment the composition is formulated as a gel or lotion comprising the DNase that has endonuclease or exonuclease activity (e.g. DNase I), ATA and an anti-microbial agent.

In accordance with one embodiment bandages, gauze, wraps (crêpe rolled bandages) or other wound covering materials are infused with any of the biofilm disrupting composition disclosed herein for release of the composition from the bandage, wrap or delivery vehicle after application of the bandage, wrap or delivery vehicle to a wounded surface of mammalian skin. In one embodiment the biofilm disrupting composition comprises a thickener selected from the group consisting of methyl cellulose, hydroxypropyl methyl cellulose, hydroxyethyl cellulose, guar, hydroxyethyl guar, xanthan gum, sodium salt of cross linked polyacrylate and hyaluronic acid.

According to the claimed invention, the biofilm disrupting compositions disclosed herein further comprise an anti-microbial agent. In one embodiment the antimicrobial agent is an antibiotic. In one embodiment the antibiotic is a topical antibiotic selected from the group consisting of sulfacetamide sodium, erythromycin, silver sulfadiazine, mupirocin, bacitracin, neomycin, polymyxin, bacitracin, neomycin, polymyxin B and pramoxine.

In accordance with one embodiment any of the biofilm disrupting compositions disclosed herein can further comprise an antiseptic, optionally wherein the antiseptic is selected from the group consisting of cadexomer iodine, povidone iodine, cetrimide, benzalkonium chloride, chlorhexidine gluconate, polyhexanide, hydrogen peroxide, octenidine dihydrochloride, diamidines, silver compounds and zinc salts.

In accordance with one embodiment any of the biofilm disrupting compositions disclosed herein can further comprise an amylase, cellulase, or a protease, or mixtures thereof.

In accordance with one embodiment any of the biofilm disrupting compositions disclosed herein can be used to adversely affect an established biofilm, or prevent the establishment or reoccurrence of a biofilm. In accordance with one embodiment this comprises the steps of contacting the biofilm, or a site at risk of formation of a biofilm, with a composition as defined in the appended claims.

In one embodiment a non-therapeutic method is provided for disrupting a biofilm, and more particularly a hyperbiofilm, as described in claim 7, as well as a biofilm disrupting composition as defined in any one of claims 1 to 6 for use in a method of disrupting a biofilm, as described in claim 9.

The infection to be treated may be a biofilm infection and the biofilm infection may be present in a chronic wound. In embodiments of the invention, the composition may be administered topically. The biofilm infection may be a bacterial biofilm infection, such as a *Pseudomonas aeruginosa* biofilm infection that includes a rugose small colony variant (RSCV) of *P. aeruginosa.* The DNA specific endonuclease may be a protease-free DNase I and the inhibitor of protein-nucleic acid binding is aurine tricarboxylic acid. In embodiments of the invention, the composition inhibits eDNA-protein interaction in the biofilm infection.

In accordance with one embodiment a a biofilm disrupting composition as defined in any one of claims 1 to 6 for use in inhibiting the infection of wounds and/or treating chronic wound infections is provided. This comprises administering to said wound a biofilm disrupting composition according to any of the compositions disclosed herein in an amount effective to treat said wound. In one embodiment the administration of the biofilm disrupting composition is co-administered with an antimicrobial agent. In one embodiment the antimicrobial agent is an antibiotic or an antifungal agent, or a combination thereof. In one embodiment the antibiotic is selected from the group consisting of beampicillin, amoxicillin/clavulanate, metronidazole, clindamycin,erythromycin, gentamicin, vancomycin, ciproflaxin, clindamycin,tetracycline, an anxiolytic, amikacin, kanamycin, neomycin, netilmicin,streptomycin, tobramycin, teicoplanin, vancomycin, azithromycin,clarithromycin, clarithromycin, dirithromycin, erythromycin,roxithromycin, troleandomycin, amoxicillin, ampicillin, azlocillin,carbenicillin, clozacillin, dicloxacillin, flucozacillin, mezlocillin,nafcillin, penicillin, piperacillin, ticarcillin, bacitracin, colistin,polymyxin B, ciprofloxacin, enoxacin, gatifloxacin, levofloxacin,lomefloxacin, moxifloxacin, norfloxacin, oflazacin, trovafloxacin,mafenide, sulfacetamide, sulfamethizole, sulfasalazine, sulfisoxazole, trimethoprim, cotrimoxazole, demeclocycline, soxycycline, minocycline,doxycycline, and oxytetracycline. In one embodiment the antibiotic is a topical antibiotic selected from the group consisting of sulfacetamide sodium, erythromycin, silver sulfadiazine, mupirocin, bacitracin, neomycin, polymyxin, bacitracin, neomycin, polymyxin B and pramoxine. According to the claimed invention, the biofilm disrupting composition is formulated to comprise a DNase that has endonuclease or exonuclease activity, ATA and an antimicrobial agent, optionally wherein the nuclease is DNase I.

The clinical rugose small colony variant (RSCV) of *Pseudomonas aeruginosa* is hyperactive in biofilm formation during chronic infection. Under laboratory conditions, emergence of some RSCVs relies on loss-of-function mutations in the methylesterase-encoding gene wspF. Such mutations in RSCV result in constitutive overexpression of both Pel and Psl exopolysaccharides. RSCVs are difficult to eradicate and are responsible for recurrent or chronic infections. In biofilms, RSCVs are deeply embedded in self-produced hydrated EPSs. The Psl and Pel exopolysaccharides, together with extracellular DNA (eDNA), serve as structural components of the biofilm matrix.

*Pseudomonas aeruginosa* biofilms represent a major threat to healthcare. Rugose small colony variant (RSCV) of *P. aeruginosa* (PAO1) is frequently isolated from chronic infections. Loss of the methylesterase-encoding gene *wspF* causes the isogenic RSCV strain of PAO1 (PAO1ΔwspF) to form robust biofilm. RSCV biofilms are highly resistant to antibiotics and host defenses. RSCV consists of a unique blend of structurally diverse sub-populations. Scanning transmission electron microscopy (STEM) tomography of PAO1ΔwspF revealed two different bacterial subpopulations that display distinct spatial organization in biofilm aggregates. Comparative analyses of the structure of PAO1 and PAO1ΔwspF biofilms revealed unique structural characteristics of the PAO1ΔwspF extracellular polymeric substance (EPS). Unlike PAO1, PAO1ΔwspF biofilms exhibited the presence of smaller size extracellular DNA (eDNA). Such fragmented eDNA was responsible for higher resistance of PAO1ΔwspF biofilm to disruption by DNase I treatment. Topical addition of such low molecular weight eDNA to PAO1 enhanced biofilm formation. Inhibition of eDNA-protein interaction compromised PAO1ΔwspF biofilm formation.

In accordance with one embodiment a method is provided for disrupting the biofilm matrix of RSCV, or adversely affecting an established RSCV biofilm. The method comprises contacting the RSCV biofilm with a composition according to the claimed invention.

### REFERENCE-EXAMPLE 1

The structural characteristics of bacterial biofilm contribute to their pathogenicity. Diversity in the structural elements of bacterial biofilm has been of interest as insight into biofilm ultrastructure is likely to unveil novel therapeutic strategies for eradicating persistent infection. As disclosed in the following study the ultrastructure of the hyperbiofilm-producing *P. aeruginosa* RSCV strain PAO1ΔwspF was investigated with reference to its isogenic strain PAO1.

*P. aeruginosa* RSCVs cause persistent infection, because they are recalcitrant to antibiotics and host immune cells. Scanning transmission electron microscopy (STEM) tomography is powerful in unveiling the structural characteristics with nanometer-scale spatial resolution and insight gained from STEM imaging and tomography has led to novel mechanistic hypothesis. Specifically applicant found that inhibition of EPS protein-eDNA interaction is a specifically effective strategy to dismantling biofilms formed by RSCVs.

Scanning transmission electron microscopy (STEM) imaging and tomography offers the opportunity to investigate the ultrastructure of aggregated macromolecular complexes in the EPS with nanometer scale spatial resolution. In STEM, a focused electron beam (<1nm diameter) scanned across the specimen and the transmitted signal is collected pixel-by-pixel. Images collected as a function of sample rotation angle (with respect to the electron beam direction) enable 3D reconstruction.

In STEM images of non-crystalline materials recorded using a high-angle angular dark field (HAADF) detector, mass thickness is the dominant contrast mechanism. A region that has higher mass density or is thicker will scatter more electrons. Consequently, the HAADF-STEM signal will be more intense, and the region will exhibit "white" contrast. Unlike conventional confocal microscopy, STEM imaging of PAO1 and PAO1ΔwspF biofilms revealed two distinct subpopulations that were uniquely organized in the hyperbiofilm strain (PAO1ΔwspF) compared with that in the wild-type (PAO1) variety. Two distinct subpopulations, "white" and "grey" contrast, were noted in the STEM-HAADF. For purposes of the present disclosure, these subpopulations are referred to as bacteria_{white} and bacteria_{gray}, respectively.

On the basis of these observations, a density gradient centrifugation approach was developed to separate the two different subpopulations of bacteria: bacteria_{white} and bacteria_{grey}. In the PAO1 biofilm, bacteria_{white} and bacteria_{gray} were homogenously distributed throughout the biofilm. In contrast, the PAO1ΔwspF biofilm showed a segregated spatial distribution such that bacteria_{white} were found at the apical and bacteria_{gray} at the basal regions of the biofilm. Thus, bacteria_{white} were localized toward the air interface, whereas bacteria_{gray} were more proximal to the nutrient-supplying basal interface. As the microtomed specimens have negligible variations in thickness, the effect of thickness on the scale of contrast variations can be discounted. Thus the differences between bacteria_{white} and bacteria_{gray} are attributed to their mass-density difference. On the basis of these observations, a density gradient centrifugation approach was developed to separate the two different subpopulations of bacteria: bacteria_{white} and bacteria_{gray}. The pellet obtained after density gradient centrifugation was designated as bac_{heavy} and the supernatant as bac_{light}. STEM-HAADF images showed that the bac_{heavy} fraction was predominantly comprised of bacteria_{white}. The bac_{light} fraction was predominantly bacteria_{gray}. PAO1Δ*wspF* biofilm bacteria were in strict adherence to these rules validating our notion that the bacteria_{white} have higher mass density than the bacteria_{gray}. The separation of bacteria_{white} and bacteria_{gray} from PAO1 biofilm cells after density gradient centrifugation was not as efficient as that in the PAO1ΔwspF biofilm cells. Although the predominance of bacteria_{white} was indeed more in the bac_{heavy} fraction of PAO1 biofilm, some were present in the bac_{light} fraction as well.

**In** an effort to investigate functional contrasts between bac_{light} and the bac_{heavy}, cellular respiration was studied using a real-time prokaryotic respiration assay (SeaHorse XFe extracellular flux analyzer) (Lobritz et al., 2015 Proc. Natl. Acad. Sci.US A 172, 8173-8180). Compared with bac_{heavy}, bac_{light} showed elevated oxygen consumption indicative of higher aerobic metabolism of biofilm bacteria localized toward the nutrient interface. Respiration of bac_{heavy} was detected, compared with heat-killed bacteria, indicating that bac_{heavy} were metabolically less active, but not dead (see Fig. 1).

**In** another experimental system for use in studying intact biofilms, the DNA-intercalating dye propidium iodide (PI) stained abundantly toward the air interface in PAO1ΔwspF biofilms. Taken together, **PI** stain as well as cellular respiration leads to the conclusion that bacteria_{white} have reduced metabolic capacity but have much higher abundance of eDNA in their EPS microenvironment. Thus, this work draws a direct connection between the structural elements and functional properties of bacterial subpopulations within the same biofilm. Importantly, in the hyperbiofilm RSCV, the basal subpopulation proximal to the nutrient interface was metabolically hyperactive compared with the same subpopulation in the wild-type strain. Such observation may be explained by the finding that in PAO1, the basal hypermetabolic bacteria_{gray} population is somewhat diluted by the presence of few hypometabolic bacteria_{white} cells. However, in PAO1ΔwspF biofilm, the basal subpopulation consists of a homogeneous population of hypermetabolic bacteria_{gray} cells. PAO1ΔwspF Release Segmented eDNA in Biofilm

**In** PAO1, lysis of a subpopulation of bacteria contributes to the eDNA pool, which in turn facilitates the self-organization of biofilm structures. **In our** experimental system investigating PAO1, consistent findings were noted. Lysed PAO1 indeed contributed to eDNA as observed from live cell imaging with cell-impermeant DNA-binding dye TOTO-1 that specifically stains eDNA. STEM imaging revealed the products of bacterial lysis within the PAO1 biofilm. **In** PAO1ΔwspF biofilm, however, remnants of lysed bacteria were rarely evident. Further investigation into the source of eDNA in EPS of PAO1Δ*wspF* revealed extrusion of DNA from live cells into the extracellular compartment. Such process was not associated with bacterial lysis as reported for PAO1. Because **PI** stains both eDNA and intracellular DNA of bacteria with compromised wall integrity, the **PI** data from PAO1ΔwspF biofilm alone is inadequate to draw any conclusion. To address this, live cell imaging with TOTO-1 and **PI** was performed in PAO1ΔwspF. Unlike heat-killed PAO1ΔwspF, evidence of PI⁻ bacteria showing TOTO-1 staining supports the fact that PAO1ΔwspF possess a distinct mechanism of extruding DNA without undergoing lysis as commonly seen in PAO1.

HAADF-STEM imaging and tomography provides unprecedented insight into the ultrastructure of a wild-type and its corresponding hyperbiofilm variant. **In** PAO1, heterogeneous mixture of globular debris was abundant in EPS. **In** contrast, EPS of PAO1ΔwspF biofilm showed thread-like structures associated with vesicular structures. The observed heterogeneous mixture of globular debris in PAO1, which appears white in HAADF-STEM images, was sensitive to DNase I treatment supporting the notion that it is eDNA. **In** PAO1, DNase I treatment completely eliminated all globular debris-like structures and compromised the structural integrity of the biofilm to a point where fixation of samples for HAADF-STEM imaging was challenging. **In** the few cases wherein samples could be processed, distorted morphology of individual PAO1 bacteria were observed. **In** cases wherein the structural integrity of the PAO1 biofilm was completely lost, the sloughed off samples were pelleted by centrifugation. Such pellets were processed for STEM imaging as described. Of note, the resulting images provided information on the content of each sample and not on its structure. Elimination of the globular debris-like structures following DNase I treatment was evident. This observation further supports the conclusion that the heterogeneous mixture of globular debris was eDNA. However, unlike the PAO1 biofilm, the *PAO1ΔwspF* biofilm was resistant to DNase I treatment (see Fig. 3A). Following DNase I treatment, PAO1ΔwspF biofilm retained appreciable structural integrity including some DNase I-resistant structures in the EPS. These retained structures associated with aggregates of vesicular structures only in the EPS of PAO1ΔwspF. Thus, there are clear differences in the structural characteristics of the biofilm of the wild-type and its variant.

### eDNA in PAO1ΔwspF Biofilm Represented Only Part of PAO1ΔwspF Genome DNA

Explosive lysis of *P. aeruginosa* has been reported to contribute eDNA to EPS of PAO1. Thus, whole-genomic DNA was expected in the EPS of a PAO1 biofilm. Interestingly, abundance of eDNA in the biofilm of PAO1 and PAO1ΔwspF was comparable (Figs. 3B and 3C) Our findings on PAO1, the wild-type reference strain of this study, showed that indeed the eDNA of PAO1 biofilm was intact and represented the entire genome whereas the eDNA of PAO1ΔwspF biofilm was mostly fragmented (Fig. 2) with size range of 25-400 bp. In the context of evidence on DNA extrusion from live PAO1ΔwspF bacteria and lack of entire genome representation in the eDNA it is concluded that these hyperbiofilm bacteria are capable of contributing eDNA to the extracellular compartment without necessarily having to go through the suicidal path of explosive lysis. In this process, abundant eDNA is deposited as needed for biofilm structure. In the context of hyperbiofilm PAO1ΔwspF bacteria, an important question that arises is whether the eDNA is fragmented within the cell and then exported or whether intact DNA exported by the live cell undergoes fragmentation in the extracellular space. In the current work, next-generation sequencing of eDNA from the PAO1 biofilm was identical to that from the PAO1 genome, supporting the previously reported observation of explosive lysis of PAO1. *PAO1ΔwspF* biofilm did not follow that pattern. In this case, the eDNA showed little resemblance to the PAO1 genome. This observation becomes even more interesting considering the fact that both total eDNA content and DNase activity were comparable in the EPS of PAO1 and PAO1ΔwspF biofilms. These observations led us to test the hypothesis that unlike PAO1, hyperbiofilm-forming PAO1ΔwspF bacteria possess the unique ability to extrude DNA fragments as part of bolstering their biofilm structure.

### Interaction of Fragmented DNA with EPS Protein Results in Formation of Robust Biofilm

In the current work, addition of EPS from PAO1ΔwspF to PAO1 augmented biofilm formation (Fig. 4A & 4D). However, addition of EPS from PAO1 to PAO1ΔwspF did not influence its biofilm-forming ability (Fig. 4B). To elucidate the functional significance of EPS component eDNA in biofilm formation, intact genomic DNA was isolated from PAO1 and subjected to DNase I digestion. Addition of this fragmented DNA to PAO1 showed no significant change in bacterial growth curve when compared with addition of intact DNA to PAO1 (Fig. 4C). However, such addition of fragmented DNA accelerated biofilm formation in PAO1. Compared to addition of intact DNA, fragmented DNA showed clear enhancement of biofilm formation and thus fragmented eDNA was more effective in interacting with biofilm matrix (Fig. 4A). Most biofilm matrix proteins stain positive with SYPRO Ruby. Consistently, crystal violet assay for biofilm quantification supported the same conclusion demonstrating that fragmented DNA enhanced biofilm formation. DNA is known to possess adhesive property, which facilitates interaction with other biomolecules to ensure structural integrity of the biofilm. Observations of the current study lend credence to the notion that fragmented eDNA, as opposed to intact DNA, provides additional advantage to the process of biofilm formation. Interestingly, hyperbiofilm bacteria utilize this edge to their advantage.

Bacteria with hyperbiofilm characteristics employed fragmented eDNA to achieve better interaction with macromolecules in the EPS (Fig. 5A). To test the significance of such interaction in biofilm formation, the EPS isolated from PAO1ΔwspF biofilm was incubated with aurine tricarboxylic acid (ATA), a pharmacological inhibitor of protein-nucleic acid binding (Gonzalez et al., 1979 Biochim. Biophys. Acta 562, 534-545). ATA significantly compromised the biofilm-forming ability of PAO1 (Fig. 5B). Protein-nucleic acid binding played a significant role in biofilm formation by RSCV. However, ATA did not affect bacterial growth as evident from PAO1ΔwspF growth curve (Fig. 5C). Specifically, ATA limited protein-nucleic acid interaction in PAO1ΔwspF biofilm (Fig. 5C and Fig. 5D).

### Discussion

*P. aeruginosa* RSCVs cause persistent infection, because they are recalcitrant to antibiotics and host immune cells. This work reports the first evidence for the presence and distribution of two distinct bacterial populations, apical bacteria_{white} and basal bacteria_{gray}, in the PAO1ΔwspF biofilm. The distribution of these two distinct bacterial populations in the PAO1ΔwspF biofilm was not only morphological but also physiological.

Findings of this work demonstrate that the oxygen consumption of basal bacteria_{gray} was elevated compared with that of the apical bacteria_{white} population. These data were consistent with the previous report from the spatial distribution of *Escherichia coli* macrocolony biofilms. According to that report, bacteria in the basal region were dividing with minimal ribosomal synthesis, whereas bacteria in the apical region displayed limited cell division yet robust ribosomal synthesis. This work reports the first identification and separation of these two distinct bacterial populations.

A growing body of research now acknowledges the presence of extracellular forms of DNA and their role as important structural components of the biofilm matrix. The formation of a biofilm also relies on the structural proteins that provide the three-dimensional architectural integrity and functionality. Negatively charged eDNA interacts with positively charged proteins and polysaccharide to form the structural backbone of the bacterial biofilm. How eDNA stabilizes the *P. aeruginosa* biofilm structure and contributes to antimicrobial tolerance remains unclear. This work recognizes the fact that intact bacterial DNA presents itself as eDNA in PAO1 biofilm supporting the contention that such DNA is delivered by bacterial cell lysis. Explosive lysis of *P. aeruginosa* has been shown to be responsible for eDNA contents of biofilm. eDNA in *P. aeruginosa* is similar to whole-genome DNA. Consistently, our work reports intact eDNA in the PAO1 biofilm. Interestingly, in a PAO1ΔwspF biofilm, eDNA was mostly fragmented. Thus, whether the DNA is fragmented in the matrix or processed inside the bacteria emerges as an interesting question. That bacterial cellular DNA may be exported by live cells has been recently shown in *Staphylococcus aureus.* Genome-wide screening for genes involved in forming robust *S. aureus* biofilms identified gdpP and xdrA that are involved in the release of eDNA. Whether, unlike PAO1, viable non-lytic PAO1ΔwspF is capable of digesting part of its own DNA and extruding such digest to support the biofilm structure needs further investigation.

Consistent with the notion that eDNA provides critical support to the biofilm structure, DNase I treatment compromised PAOT1 biofilm. In contrast, the structural integrity of PAO1ΔwspF biofilm was mostly unaffected by such enzymatic treatment. After DNase I treatment, although eDNA was removed at the basal region, thread-like eDNA persisted from the middle to the apical region of the PAO1ΔwspF biofilm. Emerging studies reveal that interaction between eDNA and other EPS components may stabilize biofilm structure (Schwartz et al., 2016 Mol. Microbial. 99, 123-134). For example, pyocyanin, a metabolite of *P. aeruginosa,* interacts with eDNA enhancing bacteria cell aggregation (Das et al., 2013 PLoS One 8, e58299). In *P. aeruginosa* biofilm, negatively charged eDNA and positively charged Pel polysaccharide are cross-linked by ionic forces (Jennings et al., 2015 Proc. Natl. Acad. Sci.US A 112, 11353-11358). The Psl-eDNA fiber-like structure helps to form the biofilm skeleton in *P. aeruginosa* (Wang et al., 2015 Environ. Microbial. Rep. 7, 330--340).

Biofilms are more susceptible to antibiotics after eDNA is removed by DNase. Although DNase I treatment did not dismantle the biofilm structure of PAO1ΔwspF, it was helpful in separating bac_{light} and bac_{heavy} cells, pointing toward a potential role of eDNA in the adhesion of these cells. In *P. aeruginosa,* addition of eDNA enhances biofilm structure (Yang et al., 2009 Mol. Microbial. 74, 1380--1392). On the other hand, addition of excessive eDNA may inhibit planktonic bacteria growth and biofilm formation. In this work, cell growth of *P. aeruginosa* was not altered in the presence of digested DNA at a concentration of 100 ng/mL (Fig. 4D). Interestingly, addition of genomic DNA digest increased DNA-protein interaction and accelerated biofilm formation. Indeed, nucleoid-associated proteins are known to connect eDNA strands in *Haemophilus influenzae* biofilm (Goodman et al., 2011 Mucosal Immunol. 4, 625-637). Targeting eDNA-protein interactions disperses *Burkholderia cenocepacia* biofilms (Novotny et al., 2013 PLoS One 8, e67629). Proteomic findings of this work revealed the co-existence of higher abundance of nucleic acid-binding protein and fragmented eDNA at the apical bacteria_{white} region. Inhibition of DNA-protein interaction with ATA blunted biofilm formation by *PAO1ΔwspF.*

STEM images reported herein provide unprecedented comparative insight into the structure of prototypical *P. aeruginosa* and its isogenic RSCV strain PAO1ΔwspF. This work reports the first evidence for the presence and segregated distribution of two distinct bacterial populations, apical bacteria_{white} and basal bacteria_{gray}, in the *PAO1ΔwspF* biofilm. These bacteria were not only phenotypically different but also showed difference in oxygen consumption rate. Furthermore, resistance to DNase digestion in RSCV was attributed to the fact that the eDNA in the EPS was fragmented. The strategy to inhibit protein-DNA interaction using ATA was effective in dismantling biofilms formed by RSCV. Taken together, this work provides unprecedented visual cues into the structure of biofilm formed by *P. aeruginosa* upholding clear structural as well as functional differences between wild-type and its hyperbiofilm variant.

### Materials and Methods

**Bacterial strain.** *P. aeruginosa* prototypical strain PAO1 and its isogenic RSCV PAO1ΔwspF were used in this study. Under laboratory conditions, emergence of RSCVs relies on loss-of-function mutations in the methylesterase-encoding gene *wspF.* Cultures were routinely grown on Luria-Bertani (LB) agar or in LB broth.

***In vitro* biofilm.** *In vitro* PAO1 and PAO1ΔwspF biofilm were developed on a 10 mm polycarbonate membrane (PCM) filter as described previously. Briefly, following overnight culture in LB medium at 37°C, the bacteria were inoculated on sterile PCM filters placed on trypticase soy agar (TSA) (Catalog No: 22091, Sigma-Aldrich, USA) plates. The plates were incubated at 37°C for 24h, after which the PCMs were transferred to a new TSA agar plate. The PCM filters were kept for additional 24h for the biofilm to mature.

**Treatment of *in vitro* biofilm.** In some experiments, the 48h matured biofilm was treated with RNase free DNase I (Roche, 04716728001) for 30 min at 37°C prior to sample processing. The 1X buffer (Roche, 04716728001) without the DNase I (Roche, 04716728001) was used as vehicle control. In other set of experiments, we treated the 48h biofilm cultures with 0.5µM ATA (aurintricarboxylic acid) (A1895 Sigma) for 30 min at 37°C. For PAO1 and PAO1ΔwspF biofilm assays, a time point of 12h and 24h was chosen.

**Scanning transmission electron microscopy (STEM) sample preparation.** Biofilms were primarily fixed with 2.5% glutaraldehyde and 2% paraformaldehyde in 0.15M-cacodylate buffer. After washed three times with 0.15M-cacodylate buffer, the primarily fixed biofilms were post-fixed with 2% reduced osmium tetroxide. The biofilms were then washed with distilled water and further stained with 1% uranyl acetate. The stained samples were dehydrated in an increasing series of ethanol (30%, 50%, 70%, 80%, 90%, 2x100%) for 15 min each. After dehydration, samples were immersed in 1:0, 3:1, 1:1 and 1:3 acetone/resin for 60 minutes each and then kept in 100% resin overnight. Lastly the samples were transferred in fresh 100% resin and incubated at 65°C for 2 days to form a polymerized resin block.
90nm ultra-fine sections were cut from the resin block using a Reichert-Jung (Leica, Wetzlar, Germany) Ultracut E ultramicrotome. The thin sections were picked up with a loop and put on 400 meshes copper grids. For tomography, 500 nm thick sections were cut and put on copper grids with parallel bars. The thick sections were oriented so that the biofilm-growing base was perpendicular to the parallel bar. The copper grids with resin-embedded samples were air dried and then coated with 3nm thick amorphous carbon on both sides.

**STEM image acquisition.** Electron micrographs were collected in STEM mode on a Tecnai F20 S/TEM (Thermo Fisher Scientific, Hillsboro) with high angle angular dark field (HAADF) detector. Microscope was operated at an acceleration voltage of 200kV using Tecani Imaging and Analysis (TIA) software. Images size was 2,048×2,048 pixels. Exposure time was 25s.

**STEM Tomography and data processing.** STEM tomography was collected on the FEI probe-corrected Titan3^{™} 80-300 S/TEM (Thermo Fisher Scientific, Hillsboro). The microscope was operated at an acceleration voltage of 300kV. Images with 2,048×2,048 pixels were recorded with HAADF detector. Single-axis tilt series ranging from -65° to 65° with 1° interval steps were recorded by using the FEI Xplore3D software (Supplementary Movie 11). Sample tilting, focusing and image shift correction were controlled by Xplore3D software. STEM dynamic focus was activated to ensure areas of interest are imaged in focus even at high tilt angles. Tracking was set after exposure. Tomographic tilt series were aligned and reconstructed using IMOD software package (University of Colorado). 3D reconstruction was built by weighted back-projection method. Images were visualized using IMOD, Chimera and Avizo software's. Movies were made using Avizo software.

**Scanning electron microscopy.** Scanning electron microscopy was performed on the *in vitro* biofilm as described previously. Briefly, the biofilm on PCM filters were fixed in 4% formaldehyde / 2% glutaraldehyde solution for 48 hours at 4°C, and subsequently dehydrated in graded ethanol series. The samples were mounted on an aluminum stub and were sputter coated with gold-palladium (Au/Pd) and imaged under the scanning electron microscope (XL 30S; FEG, FEI Co., Hillsboro, OR) operating at 5 kV in the secondary electron mode.

**Immunofluorescence** staining of biofilm and confocal microscopy: Biofilms were washed three times with sterile PBS. The density and architecture of the extracellular polymeric substances (EPS), referred to here as "extracellular matrix," was stained with 100 mg/ml FITC-conjugated Hippeastrum Hybrid Amaryllis lectins. (HHA; specific for Psl) for two hours at 4°C. The biofilms were then washed and fixed with 4% paraformaldehyde. Prior to imaging, the biofilms were stained with DAPI. For detection of extracellular DNA, TOTO^{™}-1 iodide staining (ThermoFisher Scientific, Cat # T3600; dilution 1:1000) was done. Confocal microscopy was performed using Olympus FV1000 filter confocal system at 40x, N.A. 0.45 objective lens (Olympus America Inc, Melville NY). Live cell imaging was done with LSM880 laser scanning confocal microscope. For the live dead staining of the bacteria, 48h biofilms were incubated for 30 min with a solution containing Syto Green (live) and propidium iodide (dead) (Invitrogen) as per manufacture's instruction. For the study of biofilm matrix, 48h biofilms were incubated for 45min with a solution containing Film Tracer SYPRO Ruby dye (Invitrogen) as previously described, with minor modifications. SYPRO Ruby fluorescence images were acquired by Olympus FV1000 filter confocal microscope with excitation at 457nm and emission at 610nm. After z-series acquisition, a z image through the image stack, perpendicular to the substrate, was generated.

Bacterial oxygen consumption assay. The XFe96 Extracellular Flux Analyzer (Seahorse Bioscience) was used to quantitate oxygen consumption rates (OCRs) as described previously. Briefly, 48h after biofilm were disrupted and separated using density gradient centrifugation. The separated fractions were diluted to an OD₆₀₀ of ~0.3. Cells were added to XF Cell Culture Microplates pre-coated with poly-D-lysine (PDL). Cells were centrifuged for 10 min at 1,400 × g in a Multifuge x1R (M-20 rotor) to attach them to the pre-coated plates. After centrifugation, 160 µL of fresh media was added to each well.

Extracellular Polymeric Substance (EPS) isolation: EPS was isolated and purified from *in vitro* biofilm with some modifications. Briefly, 48h old *in vitro* biofilm was transferred into 500µL of PBS (phosphate buffered saline), and vortexed. Complete recovery of EPS was done by vortexing at least for three times. PCM membrane was discarded after recovery of EPS. 37.5% of formaldehyde was added into the cultured solution and incubated for 1 hour at room temperature on shaker (100 rpm). The treated solution was mixed with 1M sodium hydroxide and incubated for 3h at room temperature. This solution was centrifuged at 16,800g for 1 hour at 40°C. Supernatant was filtered through 0.2µm filter. EPS was stored at -80°C for further use. Sterility of purified EPS was checked by spreading 50µL of EPS on TSA agar plates followed by incubation at 37°C for 48 hours. The whole EPS was electrophoresed on 1% agarose gel for visualizing the EPS DNA. In some experiments, the DNA was extracted from EPS and subjected to EPRS analysis using Agilent high sensitivity D1000 tape station.

**Bacterial growth curve.** *P. aeruginosa* PAO1 and PAO1ΔwspF were cultured in Luria-Burtani (LB) medium at 37°C in round bottom tubes with continuous shaking at 300 rpm. The optical density of the media at 600 nm was recorded over different time points and plotted graphically.

**Crystal violet assay for biofilm quantification.** *P. aeruginosa* PAO1 and PAO1ΔwspF were cultured in Luria-Bertani (LB) medium at 37°C in pre-sterilized 96 well flat bottom polystyrene micro-titre plates in triplicates as described previously. Briefly, biofilms were fixed with 99% methanol. The plates are washed twice with PBS and air-dried. Then, 100 µl of crystal violet solution (0.1%) was added to all wells and incubated for 15 mins. The excess crystal violet was removed and plates were washed twice, air dried and finally dissolved in 30% acetic acid. Biofilm growth was monitored in terms of O.D₅₇₀ nm using micro plate reader.

**Genomic DNA isolation and agarose gel electrophoresis.** Genomic DNA from PAO1 and PAO1ΔwspF was isolated by GenElute^{™} Bacterial Genomic DNA Kit, Sigma-Aldrich, USA following manufacturer's instructions. 1.5 mL of 10⁶ CFU mL⁻¹ logarithmic bacterial broth culture were taken for genomic DNA isolation. The bacterial cells were pelleted by centrifuging the tube at 12,000-16,000 g for 2 min. The pellet was resuspended in 180 µL of lysis solution followed by gentle vortex. 20 µL of RNase A was added to the solution and incubated for 2 min at room temperature. 20 µL of proteinase K was added to the solution and incubated at 55°C for 30 min. 500 µL of column preparation solution was added to each column and centrifuged at 12000 g for 1 minute. 200 µL of ethanol was added to the cell lysate and mixed by vortexing for 10 s. The entire solution was transferred into the column and centrifuged at 6500 g for 1 min. The flow though was discarded and the column was rinsed with 500 µL of wash solution 1. The column was further washed with wash solution and centrifuged at 12000-16000 g for 3 min. 200 µL of elution buffer was added to the column and incubated for 5 min at room temperature. Genomic DNA was eluted following centrifugation of the column at 6500 g for 1 min. Further the genomic DNA was visualized on 0.8% agarose gel and analyzed by EPRS using Agilent genomic DNA tape station.

**Next Generation Sequencing:** PAO1 and wspF EPS DNA samples were isolated and quality check was performed by Qubit DNA Assay Kit. All samples passed internal quality control. The samples were subjected to fragmentation, adaptor addition, with final QC by Agilent 2100 Bioanalyzer and real-time PCR quantification. Whole Genome Sequencing (8 Million reads, 2x75bp, PE) was performed. The reads were first trimmed for adaptor sequences and error corrected. Genome assembly was performed using SPAdes. Genomic DNA of PAO1 and WspF were also sequenced and compared with PAO1 reference sequence (accession number: NC_002516) showing high synteny with the reference sequence (Supplementary Figure 1,2), indicating the assembly quality was adequate for subsequent analysis. Coverage analysis of each genomic region was performed. The average coverage for each EPS DNA was found to be around 300x. Read coverage was then compared between PAO1 EPS and wspF EPS sample.

**DNA digestion.** The genomic DNA isolated from PAO1 and PAO1ΔwspF strains were subjected to DNA digestion using RNase free DNase I (Roche, 04716728001) for 30 min at 37°C. The DNA was purified to remove the DNase I and 500 ng of either this digested DNA or intact DNA (without DNase I treatment and purification) was added to the bacterial culture on PCM.

**Density gradient centrifugation of *in vitro* biofilm of PAO1 and *ΔwspF.*** 48h *in vitro* biofilm of PAO1 and PAO1ΔwspF were gently vortexed in 1 ml sterile PBS for 30s to make homogenous mixture. 20 ml Ficoll (Ficoll^{®} Paque Plus, GE17-1440-03 SIGMA) was taken in a 50 ml centrifuge tube. The bacterial suspension was slowly poured on the Ficoll and the tube was centrifuged at 1800 g for 20 min. The supernatant and pellet were taken separately in new tubes. The supernatant was centrifuged at 12,000 g for 10 min at 4°C to collect the bacteria. Bacteria obtained from both supernatant and pellets were washed three times with sterile PBS. The bacterial pellet was then immediately processed for protein isolation. The total protein concentration was quantitated using BCA assay (Pierce, # 23228).

**Statistical analysis.** Samples were coded and data analysis was performed in a blinded fashion. Data were reported as mean ± SD. All experiments were performed at least three times. Student's t test (two-tailed) was used to determine significant differences. Comparisons among multiple groups were tested using analysis of variance (ANOVA). p<0.05 was considered statistically significant.

## Claims

1. A biofilm disrupting composition comprising
a DNase that has endonuclease or exonuclease activity;
aurine tricarboxylic acid (ATA) that disrupts the binding of nucleic acids to proteins; an anti-microbial agent; and
a pharmaceutically acceptable carrier.

2. The composition of claim 1, wherein the DNase is selected from the group consisting of Deoxyribonuclease I (DNase I), Deoxyribonuclease II (DNase II), Deoxyribonuclease **III** (DNase III), micrococcal nuclease, and a recombinant DNase.

3. The composition of claim 2, wherein the nuclease is DNAse I.

4. The composition of any one of claims 1-3, wherein the composition is formulated as an ointment, a gel, a liquid, an aerosol, a mist, a film, an emulsion, or a suspension.

5. The composition of any one of claims 1-4, wherein the antimicrobial agent is an antibiotic.

6. The composition of any one of claims 1-5, further comprising an amylase, cellulase, or a protease, or mixtures thereof.

7. A non-therapeutic method of disrupting a biofilm, said method comprising contacting the biofilm with the biofilm disrupting composition of any one of claims 1-6.

8. The method according to claim 7, wherein the composition is a topical formulation that is applied directly to a surface comprising a biofilm.

9. A biofilm disrupting composition according to any one of claims 1 to 6 for use in a method of disrupting a biofilm, wherein the biofilm disrupting composition is a topical formulation that is applied directly to a surface comprising the biofilm, wherein the surface is skin tissue.

10. A biofilm disrupting composition according to any one of claims 1-6 for use in inhibiting the infection of wounds and/or treating chronic wound infections.

11. The biofilm disrupting composition for use according to claim 10, wherein the biofilm disrupting composition comprises a topical antibiotic selected from the group consisting of sulfacetamide sodium, erythromycin, silver sulfadiazine, mupirocin, bacitracin, neomycin, polymyxin, bacitracin, neomycin, polymyxin B and pramoxine.

## Patentansprüche

1. Biofilmzerstörungszusammensetzung, umfassend
eine DNase, die Endonuklease- oder Exonuklease-Aktivität aufweist;
Aurintricarbonsäure (ATA), welche die Bindung von Nukleinsäuren an Proteine zerstört; ein antimikrobielles Mittel; und
einen pharmazeutisch annehmbaren Träger.

2. Zusammensetzung nach Anspruch 1, wobei die DNase ausgewählt ist aus der Gruppe bestehend aus Desoxyribonuklease I (DNase I), Desoxyribonuklease II (DNase II), Desoxyribonuklease III (DNase III), Mikrokokken-Nuklease und einer rekombinanten DNase.

3. Zusammensetzung nach Anspruch 2, wobei die Nuklease DNAse I ist.

4. Zusammensetzung nach einem der Ansprüche 1-3, wobei die Zusammensetzung als eine Salbe, ein Gel, eine Flüssigkeit, ein Aerosol, ein Nebel, ein Film, eine Emulsion oder eine Suspension formuliert ist.

5. Zusammensetzung nach einem der Ansprüche 1-4, wobei das antimikrobielle Mittel ein Antibiotikum ist.

6. Zusammensetzung nach einem der Ansprüche 1-5, ferner umfassend eine Amylase, Cellulase oder eine Protease oder Mischungen davon.

7. Nichttherapeutisches Verfahren zum Zerstören eines Biofilms, wobei das Verfahren Kontaktieren des Biofilms mit der Biofilmzerstörungszusammensetzung nach einem der Ansprüche 1-6 umfasst.

8. Verfahren nach Anspruch 7, wobei die Zusammensetzung eine topische Formulierung ist, die direkt auf eine Oberfläche aufgetragen wird, die einen Biofilm umfasst.

9. Biofilmzerstörungszusammensetzung nach einem der Ansprüche 1 bis 6 zur Verwendung in einem Verfahren zum Zerstören eines Biofilms, wobei die Biofilmzerstörungszusammensetzung eine topische Formulierung ist, die direkt auf eine Oberfläche aufgetragen wird, die den Biofilm umfasst, wobei die Oberfläche Hautgewebe ist.

10. Biofilmzerstörungszusammensetzung nach einem der Ansprüche 1-6 zur Verwendung beim Hemmen der Infektion von Wunden und/oder Behandeln von chronischen Wundinfektionen.

11. Biofilmzerstörungszusammensetzung zur Verwendung nach Anspruch 10, wobei die Biofilmzerstörungszusammensetzung ein topisches Antibiotikum ausgewählt aus der Gruppe bestehend aus Sulfacetamidnatrium, Erythromycin, Silbersulfadiazin, Mupirocin, Bacitracin, Neomycin, Polymyxin, Bacitracin, Neomycin, Polymyxin B und Pramoxin umfasst.

## Revendications

1. Composition de perturbation de biofilm comprenant
une DNase présentant une activité endonucléase ou exonucléase ;
l'acide aurine-tricarboxylique (ATA) qui perturbe la liaison des acides nucléiques aux protéines ;
un agent antimicrobien ; et
un support pharmaceutiquement acceptable.

2. Composition de la revendication 1, dans laquelle la DNase est choisie dans le groupe constitué par la désoxyribonucléase I (DNase I), la désoxyribonucléase II (DNase II), la désoxyribonucléase III (DNase III), la nucléase micrococcique et une DNase recombinante.

3. Composition de la revendication 2, dans laquelle la nucléase est la DNAse I.

4. Composition de l'une quelconque des revendications 1 à 3, dans laquelle la composition est formulée sous la forme d'une pommade, d'un gel, d'un liquide, d'un aérosol, d'un brouillard, d'un film, d'une émulsion ou d'une suspension.

5. Composition de l'une quelconque des revendications 1 à 4, dans laquelle l'agent antimicrobien est un antibiotique.

6. Composition de l'une quelconque des revendications 1 à 5, comprenant en outre une amylase, une cellulase ou une protéase ou des mélanges de celles-ci.

7. Procédé non thérapeutique de perturbation d'un biofilm, ledit procédé comprenant la mise en contact du biofilm avec la composition de perturbation de biofilm de l'une quelconque des revendications 1 à 6.

8. Procédé selon la revendication 7, dans lequel la composition est une formulation topique qui est appliquée directement sur une surface comprenant un biofilm.

9. Composition de perturbation de biofilm selon l'une quelconque des revendications 1 à 6, destinée à être utilisée dans un procédé de perturbation d'un biofilm, dans laquelle la composition de perturbation de biofilm est une formulation topique qui est appliquée directement sur une surface comprenant le biofilm, dans laquelle la surface est un tissu cutané.

10. Composition de perturbation de biofilm selon l'une quelconque des revendications 1 à 6, destinée à être utilisée pour inhiber l'infection de plaies et/ou traiter des infections chroniques de plaies.

11. Composition de perturbation de biofilm destinée à être utilisée selon la revendication 10, dans laquelle la composition de perturbation de biofilm comprend un antibiotique topique choisi dans le groupe constitué par le sulfacétamide sodique, l'érythromycine, la sulfadiazine d'argent, la mupirocine, la bacitracine, la néomycine, la polymyxine, la bacitracine, la néomycine, la polymyxine B et la pramoxine.
